# EUROPÄISCHE PATENTANMELDUNG

(11) **EP 4 763 114 A1**
(43) Veröffentlichungstag der Anmeldung: **24.06.2026**
(21) Anmeldenummer: 25220639.6
(22) Anmeldetag: 04.12.2025
(51) Int. Cl.: A61B 17/80

(54) **OSTEOSYNTHESEPLATTE**

(30) Priorität: 19.12.2024 DE 102024138941
(71) Anmelder: Karl Leibinger Asset Management GmbH & Co. KG, 78570 Mühlheim an der Donau (DE)
(72) Erfinder: Leibinger, Ralf, 78570 Mühlheim an der Donau (DE); Reinauer, Frank, 78576 Emmingen-Liptingen (DE); Aberle, Steffen, 78126 Königsfeld (DE)
(74) Vertreter: Schwamberger, Martin

(57) **Zusammenfassung**

Osteosyntheseplatte (OP), welche für eine Fixierung von Knochenfragmenten (K1, K2) zur Wiederherstellung eines Knochens (K), insbesondere einer Mandibula, vorgesehen ist, umfassend einen Plattenkörper (P), wobei der Plattenkörper (P) zumindest einen Brückenabschnitt (BU) aufweist, welcher einem Überbrücken eines zwischen den Knochenfragmenten verlaufenden Spalts (S) dient, wobei der Plattenkörper (P) mit Befestigungsabschnitten (BA1, BA2) ausgestattet ist, denen jeweils mindestens ein Befestigungspunkt (BP1, BP2) zugeordnet ist, wobei der mindestens eine Befestigungspunkt (BP2) bei zumindest einem der Befestigungsabschnitte (BA1, BA2) an einem Inselabschnitt (IA) ausgestaltet ist, welcher mit dem jeweils zugehörigen Befestigungsabschnitt (BA2) ausschließlich über Federsegmente (FS1, FS2) verbunden ist, welche eine Relativverschiebung des jeweiligen Befestigungsabschnitts (BA2) und des jeweiligen Inselabschnitts (IA) zueinander elastisch federnd zulassen, wobei zumindest eines der Federsegmente (FS1, FS2) so ausgerichtet ist, dass bei einer Belastung (B) in einer Hauptbelastungsrichtung des Knochens eine Relativverschiebung zwischen dem Inselabschnitt (IA) und dem diesem Inselabschnitt (IA) zugeordneten Befestigungsabschnitt (BA2) stattfindet, bei welcher sich eine Distanz des Inselabschnitts (IA) zu dem zumindest einen Brückenabschnitt (BU) vergrößert.

## Beschreibung

Die Erfindung betrifft eine Osteosyntheseplatte, welche für eine Fixierung von Knochenfragmenten zur Wiederherstellung eines Knochens, insbesondere einer Mandibula, vorgesehen ist, umfassend einen Plattenkörper mit einer Oberseite und einer Unterseite, auf welcher der Plattenkörper an den Knochenfragmenten aufzulegen ist. Der Plattenkörper weist zumindest einen Brückenabschnitt zum Überbrücken eines zugehörigen, zwischen den Knochenfragmenten verlaufenden Spalts auf, wobei der Plattenkörper mit beidseitig des zumindest einen Brückenabschnitts liegenden Befestigungsabschnitten ausgestattet ist. Die Osteosyntheseplatte ist an ihrer Unterseite mittels den Befestigungsabschnitten an den Knochenfragmenten zu befestigen. Dazu ist jedem Befestigungsabschnitt mindestens ein Befestigungspunkt zugeordnet, wobei der mindestens eine Befestigungspunkt bei zumindest einem der Befestigungsabschnitte an jeweils einem Inselabschnitt ausgestaltet ist. Der Inselabschnitt ist mit dem jeweils zugehörigen Befestigungsabschnitt ausschließlich über Federsegmente verbunden, welche eine Relativverschiebung des jeweiligen Befestigungsabschnitts und des jeweiligen Inselabschnitts elastisch federnd zueinander zulassen.

Bei der Osteosynthese werden im Rahmen eines operativen Eingriffs zwei oder mehr Knochenfragmente miteinander verbunden, um ein Zusammenwachsen der Knochenfragmente und damit eine Wiederherstellung des jeweiligen Knochens zu erreichen. Ziel der Osteosynthese ist dabei eine Stabilisierung der Knochenfragmente zueinander, wobei diese Stabilisierung dabei in einer korrekten Stellung und somit ggf. unter Korrektur von Fehlstellungen erfolgen soll. Neben einer Fixierung über Draht oder Schrauben kommen je nach Anwendungsbereich auch Osteosyntheseplatten zur Anwendung, wobei die jeweilige Osteosyntheseplatte dabei im Bereich eines jeweiligen Spalts zwischen den Knochenfragmenten auf den jeweiligen Knochen aufgelegt und jeweils an den miteinander zu verbindenden Knochenfragmenten befestigt wird.

Neben einer Stabilisierung von Knochenfragmenten nach einer Knochenfraktur kommen Osteosyntheseplatten teilweise auch dann zur Anwendung, wenn bei einem Knochen ein an anderer Stelle reseziertes Knochensegment an einer vorhandenen Fehlstelle einzusetzen und für eine Wiederherstellung des Knochens zu stabilisieren ist. So kann eine derartige Fehlstelle beispielsweise aufgrund einer notwendigen Entfernung eines Knochenteils nach einer Tumorerkrankung entstanden sein.

Hinsichtlich eines Heilungsprozesses des jeweiligen Knochens hat es sich als vorteilhaft erwiesen, wenn die Knochenfragmente des wiederherzustellenden Knochens nicht starr über die jeweilige Osteosyntheseplatte miteinander verbunden sind, sondern über die jeweilige Osteosyntheseplatte Mikrobewegungen der Knochenfragmente zueinander ermöglicht werden. Um diese Mikrobewegungen zu ermöglichen, werden die Befestigungspunkte auf Seiten zumindest eines der zu verbindenden Knochenfragmente über Federsegmente angebunden.

Die WO 2023/057477 A1 beschreibt ein Plattenimplantat zur Überbrückung eines Frakturspalts mit einer Längsachse in Richtung der größten Ausdehnung des Plattenimplantats und einer Mehrzahl von Bohrungen, welche voneinander beabstandet in Richtung der Längsachse angeordnet sind. Dabei ist eine elastische Längsdehnungseinrichtung vorhanden, welche zumindest zwischen zwei Bohrungen vorgesehen ist. Die Längsdehnungseinrichtung ist derart gestaltet, dass das Plattenimplantat eine erhöhte Dehnbarkeit in Richtung der Längsachse bei gleichzeitig hoher Biege- und Torsionssteifigkeit aufweist. Dadurch soll ein gewisses Maß an Längsbewegung im Frakturspalt ermöglicht werden, um die Knochenheilung zu stimulieren.

Ausgehend vom vorstehend beschriebenen Stand der Technik ist es nun die Aufgabe der vorliegenden Erfindung eine Osteosyntheseplatte zu schaffen, bei deren Verwendung bei der Wiederherstellung eines Knochens ein Heilungsprozess des Knochens weiter verbessert wird.

Diese Aufgabe wird ausgehend vom Oberbegriff des Anspruchs 1 in Verbindung mit dessen kennzeichnenden Merkmalen gelöst. Die hierauf folgenden, abhängigen Ansprüche geben jeweils vorteilhafte Weiterbildungen der Erfindung wieder. Weitere vorteilhafte Ausgestaltungen ergeben sich aus der Beschreibung sowie aus den Figuren.

Gemäß der Erfindung umfasst eine Osteosyntheseplatte einen Plattenkörper mit einer Oberseite und einer Unterseite, auf welcher der Plattenkörper an den Knochenfragmenten aufzulegen ist. Der Plattenkörper weist zumindest einen Brückenabschnitt auf, welcher einem Überbrücken eines zugehörigen, zwischen den Knochenfragmenten verlaufenden Spalts dient. Ferner ist der Plattenkörper mit beidseitig des zumindest einen Brückenabschnitts liegenden Befestigungsabschnitten ausgestattet, an welchen auf der Unterseite Befestigungen der Osteosyntheseplatte seitens der Knochenfragmente vorzunehmen sind und denen dazu jeweils mindestens ein Befestigungspunkt zugeordnet ist. Der mindestens eine Befestigungspunkt ist bei zumindest einem der Befestigungsabschnitte an einem Inselabschnitt ausgestaltet, welcher mit dem jeweiligen zugehörigen Befestigungsabschnitt des Plattenkörpers ausschließlich über Federsegmente verbunden ist, welche eine Relativverschiebung des jeweiligen Befestigungsabschnitts und des jeweiligen Inselabschnitts zueinander elastisch federnd zulassen.

Die erfindungsgemäße Osteosyntheseplatte ist für eine Fixierung von Knochenfragmenten zur Wiederherstellung eines Knochens vorgesehen. Diese Wiederherstellung des Knochens kann dabei im Rahmen der Erfindung zum einen derartig vorliegen, dass über die erfindungsgemäße Osteosyntheseplatte Knochenfragmente aneinander fixiert werden, die sich aufgrund einer Knochenfraktur des Knochens gebildet haben. Somit werden in diesem Fall die dem wiederherzustellenden Knochen zugeordneten Knochenfragmente über die Osteosyntheseplatte zueinander fixiert und stabilisiert. Zum anderen wird im Sinne der Erfindung als Wiederherstellung eines Knochens aber auch angesehen, dass eine Fehlstelle eines Knochens mit einem Knochensegment geschlossen wird, welches zu diesem Zweck bei einem anderen Knochen reseziert worden ist. Dementsprechend wird der jeweilige Knochen in diesem Fall aus eigenen Knochenfragmenten und einem anderen Knochen zugehörigen Knochenfragment wiederhergestellt. Die erfindungsgemäße Osteosyntheseplatte dient in diesem Fall dann der Fixierung des die Fehlstelle schließenden Knochensegments mit mindestens einem der beidseitig liegenden Knochensegmente. Ganz besonders bevorzugt ist die Osteosyntheseplatte für die Verwendung im Bereich des Unterkieferknochens (Mandibula) vorgesehen, wobei hierbei dann entweder eine Wiederherstellung im Rahmen einer Knochenfraktur oder im Rahmen des Schließens einer Knochenfehlstelle verwirklicht sein kann.

Die Osteosyntheseplatte ist mit einem Plattenkörper ausgestattet, welcher bevorzugt länglich ausgeführt ist, d.h. sich insbesondere zumindest überwiegend in einer Längsrichtung erstreckt. Dabei kann der Plattenkörper sich entlang einer in diese Längsrichtung verlaufenden Längsachse ausdehnen oder entlang einer in diese Längsrichtung orientierten Leitkurve verlaufend ausgestaltet sein.

Der Plattenkörper verfügt über eine Oberseite und eine Unterseite, welche bei dem Plattenkörper insbesondere einander abgewandt orientiert sind. Auf der Unterseite des Plattenkörpers findet dabei bei Verwendung der erfindungsgemäßen Osteosyntheseplatte das Auflegen des Plattenkörpers auf den Knochenfragmenten statt. Bei diesem Auflegen wird mittels des Plattenkörpers zumindest ein Spalt überbrückt, welcher zwischen den zu fixierenden Knochenfragmenten verläuft. Für dieses jeweilige Überbrücken ist der Plattenkörper mit jeweils einem Brückenabschnitt ausgestattet, wobei beidseitig dieses Brückenabschnitts Befestigungsabschnitte des Plattenkörpers liegen. An diesen Befestigungsabschnitten erfolgt bei Verwendung der Osteosyntheseplatte jeweils eine Befestigung seitens eines der über den zumindest einen Spalt getrennten Knochenfragmente, wozu jedem der Befestigungsabschnitte mindestens ein Befestigungspunkt zugeordnet ist. Bevorzugt ist der jeweilige Befestigungspunkt dabei durch jeweils eine Durchgangsöffnung gebildet, durch welche eine zugehörige Knochenschraube hindurchgeführt werden kann. Mittels der zugehörigen Knochenschraube wird dabei die Befestigung an dem jeweiligen Knochenfragment vorgenommen.

Bei zumindest einem der Befestigungsabschnitte des Plattenkörpers ist der mindestens eine vorgesehene Befestigungspunkt an einem Inselabschnitt ausgestaltet, welcher mit dem zugeordneten Befestigungsabschnitt ausschließlich über Federsegmente verbunden ist, welche den jeweiligen Inselabschnitt mit dem zugeordneten Befestigungsabschnitt koppeln. Diese Federsegmente lassen eine Relativverschiebung des Inselabschnitts zu dem zugehörigen Befestigungsabschnitt elastisch federnd zu, worunter im Sinne der Erfindung zu verstehen ist, dass sich das Federsegment bei einer Relativverschiebung, welche der Inselabschnitt und der zugehörige Befestigungsabschnitt zueinander bei Belastung vornehmen, elastisch verformt und sich bei Entlastung unter Rückbewegung in eine Ausgangsstellung wieder zurückverformt. Bevorzugt ist das Federsegment als zumindest weitestgehend linear verlaufender Federschenkel ausgestaltet.

Bevorzugt lassen die Federsegmente die Relativverschiebung zwischen Inselabschnitt und Befestigungsabschnitt nur weitestgehend parallel zu der Ober- und der Unterseite und/oder in Längsrichtung zu. Denn da in dieser Richtung aufgrund des Verlaufs des Plattenkörpers auch die Überbrückung des Spalts stattfindet, haben in diese Richtung orientierte Relativverschiebungen Änderungen des Spaltmaßes zur Folge, was sich hinsichtlich des Heilungsprozesses des wiederherzustellenden Knochens als vorteilhaft erwiesen hat.

Besonders bevorzugt steht der jeweilige Inselabschnitt auf der Unterseite des Plattenkörpers gegenüber dem zugehörigen Befestigungsabschnitt vor, wodurch bei Befestigung am jeweiligen Knochenfragment eine Auflage an dem jeweiligen Inselabschnitt bei gleichzeitigem Freiraum des Knochenfragments zu dem zugehörigen Befestigungsabschnitt stattfindet. Hierdurch kann sichergestellt werden, dass die Relativverschiebungen zwischen Inselabschnitt und Befestigungsabschnitt nicht dadurch reibungsbedingt behindert werden, dass auch der Befestigungsabschnitt auf dem ebenfalls relativ zu dem Befestigungsabschnitte zu verschiebenden Knochenfragment aufliegt.

Die Osteosyntheseplatte wird vorzugsweise mittels eines additiven Fertigungsverfahrens hergestellt, bevorzugt mittels eines 3D-Druckverfahrens. Die Osteosyntheseplatte besteht vorzugsweise aus Titan, einer Titanlegierung, aus Edelstahl oder aus einem geeigneten Polymer wie beispielsweise Polyetheretherketon (PEEK).

Die Erfindung umfasst nun die technische Lehre, dass zumindest eines der Federsegmente so ausgerichtet ist, dass bei einer Belastung in einer Hauptbelastungsrichtung des Knochens eine Relativverschiebung zwischen dem Inselabschnitt und dem diesem Inselabschnitt zugeordneten Befestigungsabschnitt stattfindet, bei welcher sich eine Distanz des Inselabschnitts zu dem zumindest einen Brückenabschnitt vergrößert.

Mit anderen Worten ist bei dem jeweiligen Inselabschnitt mindestens eines der zugeordneten Federsegmente so platziert, dass es bei einer Belastung des Knochens in einer Hauptbelastungsrichtung zu einer Relativverschiebung des jeweiligen Inselabschnitts zu dem jeweiligen Befestigungsabschnitt kommt, bei welcher Relativverschiebung sich der jeweilige Inselabschnitt von dem jeweiligen Brückenabschnitt wegbewegt.

Eine derartige Gestaltung eine Osteosyntheseplatte hat dabei den Vorteil, dass es bei Verwendung der Osteosyntheseplatte bei Belastung des Knochens in der Hauptbelastungsrichtung aufgrund der Vergrößerung der Distanz des jeweiligen Inselabschnitts zu dem Brückenabschnitt auch zu einer Vergrößerung des Spalts kommt, welcher mit dem Brückenabschnitt zwischen den Knochensegmenten überbrückt ist. Denn die die Vergrößerung der Distanz hervorrufende Relativverschiebung resultiert auch in einer Relativverschiebung der über die Osteosyntheseplatte miteinander verbundenen Knochensegmente unter Aufweitung des zwischenliegenden Spalts. Durch diese Spaltvergrößerung kann ausgeschlossen werden, dass der Spalt zwischen den Knochensegmenten zu Null wird und die Knochensegmente aneinander stoßen, was den Heilungsprozess des wiederherzustellenden Knochens erschweren würde. Bei einem Einfügen eines resezierten Knochensegments in eine Fehlstelle des wiederherzustellenden Knochens kann hierdurch außerdem ein zu kleiner oder nicht vorhandener Spalt zwischen den Knochensegmenten verhindert werden, wenn das resezierte Knochensegment im Hinblick auf die Fehlstelle mit Übergangspassung gestaltet ist. Generell werden bei Verwendung der erfindungsgemäßen Osteosyntheseplatte Mikrobewegungen zwischen den Knochenfragmenten ermöglicht, wodurch der Heilungsprozess des wiederherzustellenden Knochens angeregt wird.

Wesentlich für die Erfindung ist, dass bei dem Inselabschnitt zumindest eines der Federsegmente so orientiert ist, dass bei der Belastung des Knochens in Hauptbelastungsrichtung eine Vergrößerung des mit dem Brückenabschnitt zu überbrückenden Spalts stattfindet. Dies wird durch Hervorrufen einer entsprechend orientierten Relativverschiebung des Inselabschnitts zu dem zugeordneten Befestigungsabschnitt erreicht.

Im Sinne der Erfindung ist unter der "Belastung in Hauptbelastungsrichtung" des Knochens insbesondere eine Krafteinwirkung auf den Knochen bei dessen üblicher Beanspruchung zu verstehen. Bevorzugt findet diese Belastung dabei derartig statt, dass an dem zugehörigen Befestigungspunkt des Inselabschnitts eine resultierende Querkraft hervorgerufen wird. Im Falle der bevorzugten Ausgestaltung der Osteosyntheseplatte zur Fixierung von Knochenfragmenten zur Wiederherstellung einer Mandibula handelt es sich bei der Belastung in Hauptbelastungsrichtung insbesondere um eine über die Mandibula abzustützende Beißkraft.

Entsprechend einer möglichen Ausführungsform der Erfindung ist das zumindest eine Federsegment zur Vergrößerung der Distanz so ausgerichtet, dass dieses zumindest eine Federsegment spitzwinkelig zu einem jeweiligen Kraftvektor verläuft, welcher eine Querkraft repräsentiert, die an dem jeweiligen Befestigungspunkt des zugehörigen Inselabschnitts bei der Belastung des Knochens quer zu dem jeweiligen Befestigungsabschnitt wirkend resultiert. Bei diesem Verlauf ruft das zumindest eine Federsegment bei Einleitung der Querkraft eine Umwandlung der Querkraft in einen Längskraftanteil hervor, welcher eine von dem zugeordneten Brückenabschnitt wegweisende Relativverschiebung des Inselabschnitts zu dem jeweiligen Befestigungsabschnitt bewirkt.

Gemäß einer weiteren Ausgestaltungsmöglichkeit der Erfindung ist der Inselabschnitt über je zwei Federsegmente mit dem jeweiligen Befestigungsabschnitt verbunden. Bevorzugt sind der Inselabschnitt und die beiden zugehörigen Federsegmente durch zwei U-förmige Aussparungen gebildet, welche mit ihren Öffnungen einander zugewandt und ineinander geschachtelt in den jeweiligen Befestigungsabschnitt ausgestaltet sind. In vorteilhafter Weise lässt sich hierdurch eine einteilige Gestaltung des Inselabschnitts und der zugehörigen Federsegmente mit dem Plattenkörper erreichen, wobei hierbei geeignete Relativverschiebungen des Inselabschnitts zu dem jeweiligen Befestigungsabschnitt darstellbar sind. Die U-förmigen Aussparungen können dabei einen gleichen Verlauf aufweisen oder von ihren Verläufen her unterschiedlich gestaltet sein. Wird der Plattenkörper der Osteosyntheseplatte mittels eines additiven Fertigungsverfahrens hergestellt, so können auch die Durchbrüche bei dieser additiven Herstellung ausgestaltet worden sein. Alternativ dazu können die Durchbrüche aber auch abrasiv oder spanabhebend definiert sein.

Es ist eine weitere mögliche Ausführungsform der Erfindung, dass die Federsegmente den jeweiligen Inselabschnitt symmetrisch zu dem jeweiligen Inselabschnitt verlaufend mit dem Plattenkörper verbinden. Ganz besonders bevorzugt verlaufen die zugeordneten Federsegmente dabei punktsymmetrisch zu dem jeweiligen Befestigungspunkt des jeweiligen Inselabschnitts. Insbesondere bei Kombination mit der Variante der Erfindung, bei welcher das die Distanz vergrößernde Federsegment spitzwinkelig zu dem Kraftvektor verläuft, sorgen dann in der Folge beide punktsymmetrisch verlaufenden Federsegmente für eine die Distanz vergrößernde Kraftumleitung.

Alternativ oder bei mehreren Inselabschnitten auch ergänzend zu der vorgenannten Ausführungsform können die zugeordneten Federsegmente den jeweiligen Inselabschnitt asymmetrisch zu dem jeweiligen Inselabschnitt verlaufend mit dem Plattenkörper verbinden. Hierdurch können benachbarte Inselabschnitte bei dem jeweiligen Befestigungsabschnitt dichter aneinander gesetzt werden und/oder ein jeweils endseitig vorgesehener Befestigungspunkt näher an dem jeweiligen Ende des Befestigungsabschnitts platziert werden, wodurch sich die Anzahl an Befestigungspunkten an dem Befestigungsabschnitt steigern lässt. In Weiterbildung der vorgenannten Variante kann von den zugeordneten Federsegmenten ein Federsegment in einem unbelasteten Zustand zumindest annähernd quer zu dem jeweiligen Befestigungsabschnitt verlaufen.

Bei einer Kombination mit der Variante der Erfindung, bei welcher das die Distanz vergrößernde Federsegment spitzwinkelig zu dem Kraftvektor verläuft, wäre es im Rahmen der Erfindung auch denkbar, dass die zugeordneten Federsegmente unter voneinander abweichenden, spitzen Winkeln zu dem jeweiligen Kraftvektor und divergierend zu dem zumindest einen zugeordneten Brückenabschnitt verlaufen.

Vorzugsweise sind dem jeweiligen Befestigungsabschnitt mehrere Befestigungspunkte zugeordnet, wobei jeder der Befestigungspunkte an jeweils einem Inselabschnitt ausgestaltet ist. Insbesondere ist hierbei bei mehreren oder bei allen Inselabschnitten der mehreren Befestigungspunkte zumindest eines der zugeordneten Federsegmente so ausgerichtet, dass sich die Distanz des Inselabschnitts zu dem zugeordneten Brückenabschnitt bei der Belastung vergrößert. Dadurch kann über die mehreren Inselabschnitte gemeinsam die Vergrößerung des Spalts herbeigeführt werden. Besonders bevorzugt sind die Inselabschnitte hierbei in einer symmetrischen Anordnung an dem jeweiligen Befestigungsabschnitt ausgestaltet, d.h. die jeweiligen Inselabschnitte sind von ihrer Ausrichtung und Gestaltung her gleich an dem jeweiligen Befestigungsabschnitt ausgebildet.

Entsprechend einer weiteren Ausgestaltungsmöglichkeit der Erfindung ist von den beidseitig des zumindest einen Brückenabschnitts liegenden Befestigungsabschnitten der mindestens eine Befestigungspunkt bei dem einen Befestigungsabschnitt an dem jeweiligen Inselabschnitt ausgestaltet, wohingegen der mindestens eine Befestigungspunkt bei dem anderen Befestigungsabschnitt jeweils ortsfest an dem anderen Befestigungsabschnitt ausgebildet ist. Dadurch sind der oder die Befestigungspunkte an dem einen Befestigungsabschnitt relativ verschiebbar zu diesem Befestigungsabschnitt ausgestaltet, wohingegen der oder die Befestigungspunkte bei dem anderen Befestigungsabschnitt starr ausgestaltet sind.

Im Rahmen der Erfindung können die Befestigungspunkte auch beidseitig des zumindest einen Brückenabschnitts an Inselabschnitten ausgestaltet sein. Dementsprechend ist dann an beiden Befestigungsabschnitten eine Relativverschiebbarkeit der Befestigungspunkte gegeben. In Weiterbildung dieser Ausgestaltung ist bei den Inselabschnitten der beidseitig des Brückenabschnitts angeordneten Befestigungsabschnitte jeweils zumindest eines der zugeordneten Federsegmente die Distanz bei der Belastung vergrößernd ausgerichtet. Somit wird bei Belastung im Bereich beider Befestigungsabschnitte eine Vergrößerung des zwischen den Knochenfragmenten liegenden Spalts herbeigeführt.

Es ist eine weitere Variante der Erfindung, dass der Plattenkörper genau einen Brückenabschnitt aufweist. In diesem Fall ist der Plattenkörper der Osteosyntheseplatte also dazu ausgebildet, zwei oder mehr Knochenfragmente aneinander zu fixieren und hierbei genau einen Spalt zu überbrücken. Alternativ dazu kann der Plattenkörper mehrere Brückenabschnitte zur Überbrückung mehrerer Spalte aufweisen, wobei beidseitig des jeweiligen Brückenabschnitts liegende Befestigungsabschnitte vorgesehen sind. In diesem Fall verfügt die Osteosyntheseplatte bevorzugt über genau zwei oder mehr Brückenabschnitte und in der Folge über drei oder mehr Befestigungsabschnitte, wodurch dann mittels der Osteosyntheseplatte drei oder mehr Knochenfragmente zur Wiederherstellung des Knochens aneinander fixierbar sind.

Vorteilhafte Ausführungsformen der Erfindung, die nachfolgend erläutert werden, sind in den Zeichnungen dargestellt. Es zeigen:
- Fig. 1A und 1B: schematische Darstellungen eines Knochens mit einer hieran befestigten Osteosyntheseplatte gemäß einer Ausführungsform der Erfindung, gezeigt in unterschiedlichen Zuständen,
- Fig. 2A und 2B: schematische Ansichten eines Knochens mit einer hieran befestigten Osteosyntheseplatte entsprechend einer weiteren Ausgestaltungsmöglichkeit der Erfindung, gezeigt in unterschiedlichen Zuständen,
- Fig. 3: eine schematische Ansicht eines Teils einer Osteosyntheseplatte gemäß einer weiteren Ausführungsform der Erfindung, und
- Fig. 4: eine perspektivische Darstellung einer Osteosyntheseplatte entsprechend einer weiteren Ausgestaltungsmöglichkeit der Erfindung.

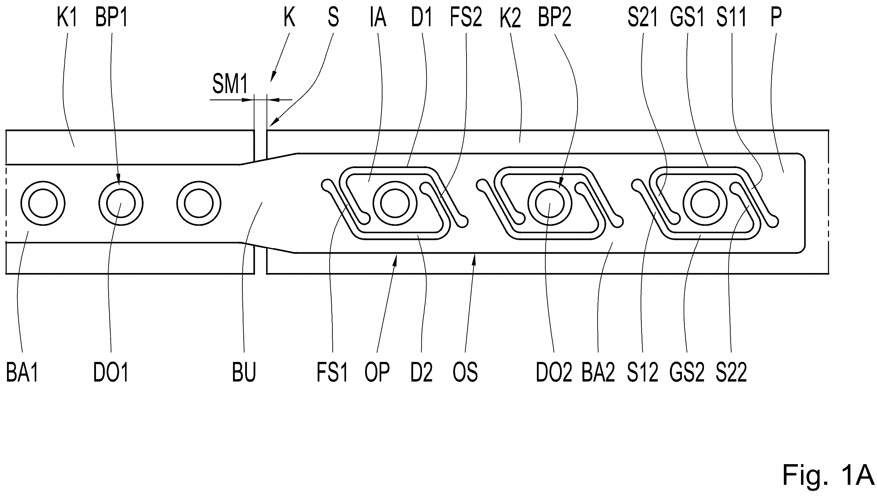

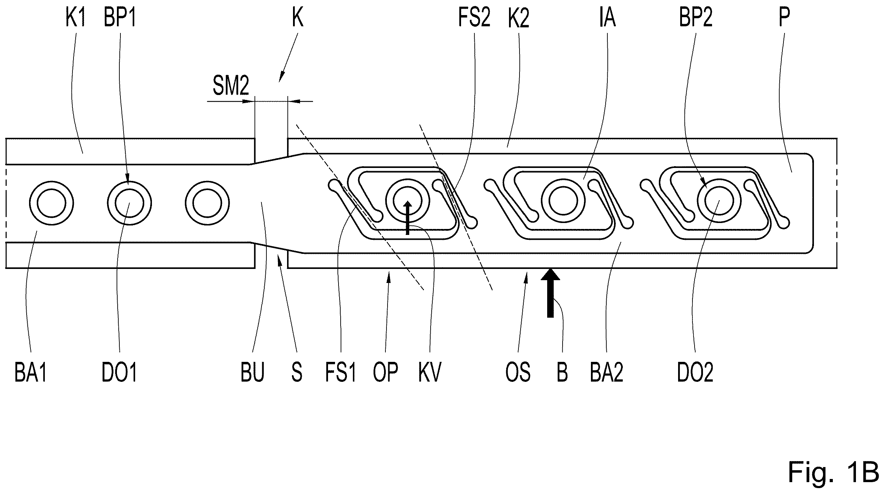

Aus den Fig. 1A und 1B gehen schematische Darstellungen eines Knochens K mit einer hieran befestigten Osteosyntheseplatte OP hervor, wobei es sich bei dem Knochen K hierbei bevorzugt um eine Mandibula handelt. Die Osteosyntheseplatte OP ist an Knochenfragmenten K1 und K2 zu befestigen, um die Knochenfragmente K1 und K2 zueinander zu stabilisieren und hierdurch ein Zusammenwachsen der Knochenfragmente K1 und K2 zur Wiederherstellung des Knochens K zu ermöglichen.

Vorliegend können sich die Knochenfragmente K1 und K2 aufgrund einer Knochenfraktur des Knochens K gebildet haben, wobei die Osteosyntheseplatte OP dann zu einem Ausheilen dieser Knochenfraktur durch die Stabilisierung der Knochenfragmente K1 und K2 zueinander vorgesehen ist. Alternativ dazu kann eines der Knochenfragmente K1 und K2 zum Schließen einer Fehlstelle des Knochens K herangezogen worden sein, beispielsweise weil an dieser Stelle ein Teil des Knochens K aufgrund einer Tumorerkrankung entfernt werden musste. Dabei handelt es sich bei dem Knochenfragment K1 bzw. K2 um einen aus einem anderen Knochen, beispielsweise dem Wadenbein, resezierten Knochenteil.

Die Osteosyntheseplatte OP weist einen länglichen Plattenkörper P auf, welcher bevorzugt aus Titan oder einer Titanlegierung besteht und insbesondere in einem additiven Herstellungsprozess, bevorzugt im 3D-Druckverfahren, gefertigt worden ist. Der Plattenkörper P weist eine in den Fig. 1A und 1B sichtbare Oberseite OS und eine gegenüberliegend angeordnete, nicht sichtbare Unterseite auf, auf welcher der Plattenkörper P an den Knochenfragmenten K1 und K2 aufgelegt ist.

Der Plattenkörper P setzt sich aus zwei Befestigungsabschnitten BA1 und BA2 sowie einem, zwischen den Befestigungsabschnitten BA1 und BA2 liegenden Brückenabschnitt BU zusammen. Dabei ist an dem Befestigungsabschnitt BA1 an der Unterseite des Plattenkörpers P eine Befestigung an dem Knochenfragment K1 vorzunehmen, wobei diese Befestigung dabei an Befestigungspunkten BP1 realisiert ist, welche dem Befestigungsabschnitt BA1 zugeordnet sind. Die Befestigungspunkte BP1 sind hierbei als Durchgangsöffnungen DO1 ausgeführt, welche sich zwischen der Oberseite OS und der Unterseite erstrecken und der Hindurchführung je einer in den Figuren nicht dargestellten Knochenschraube dienen. Dabei sind die Durchgangsöffnungen DO1 ortsfest an dem Befestigungsabschnitt BA1 ausgestaltet.

Anschließend an den Befestigungsabschnitt BA1 weist der Plattenkörper P einen Brückenabschnitt BU zur Überbrückung eines Spalts S auf. Der Spalt S liegt zwischen den Knochenfragmenten K1 und K2 vor. Anschließend an den Brückenabschnitt BU ist der Befestigungsabschnitt BA2 ausgebildet, an dem eine Befestigung des Plattenkörpers P seitens des Knochenfragments K2 vorgenommen wird. Dazu sind dem Befestigungsabschnitt BA2 Befestigungspunkte BP2 zugeordnet. Auch die Befestigungspunkte BP2 sind als Durchgangsöffnungen DO2 ausgestaltet, die zwischen der Oberseite OS und der Unterseite verlaufen und für die Hindurchführung je einer in den Figuren nicht dargestellten Knochenschraube vorgesehen sind.

Im Unterschied zu dem Befestigungsabschnitt BA1 sind die Durchgangsöffnungen DO2 bei dem Befestigungsabschnitt BA2 an Inselabschnitten IA eingebracht, die von dem Befestigungsabschnitt BA2 abgeteilt sind, indem jeder einzelne Inselabschnitt IA mit dem Befestigungsabschnitt BA2 ausschließlich über Federsegmente FS1 und FS2 verbunden ist. Die Federsegmente FS1 und FS2 lassen elastisch federnd Relativverschiebungen des jeweiligen Inselabschnitts IA und damit auch des zugehörigen Befestigungspunktes BP2 zu dem Befestigungsabschnitt BA2 zu.

Die Federsegmente FS1 und FS2 und auch der jeweils zugehörige Inselabschnitt IA sind bei der Osteosyntheseplatte OP über Durchbrüche D1 und D2 im Plattenkörper P definiert, welche U-förmig gestaltet sind und von der Oberseite OS zu der Unterseite verlaufen. Die Durchbrüche D1 und D2 sind dabei mit den Öffnungen ihrer U-Form einander zugewandt ineinander geschachtelt, wodurch neben der Abteilung des jeweiligen Inselabschnitts IA von dem Befestigungsabschnitt BA2 auch die Federsegmente FS1 und FS2 definiert werden. Die U-Formen der Durchbrüche D1 und D2 entsprechen einander, wodurch sich lineare und zueinander punktsymmetrische Verläufe der Federsegmente FS1 und FS2 ergeben. Dazu weisen beide U-Formen der Durchbrüche D1 und D2 je einen ersten, nach außen angewinkelt zu einer jeweiligen Grundseite GS1, GS2 der U-Form verlaufenden Schenkel S11, S12 auf, wobei je ein zweiter Schenkel S21, S22 bei der jeweiligen U-Form dann gegenüber der jeweiligen Grundseite GS1, GS2 nach innen angewinkelt verläuft.

In Fig. 1A ist ein unbelasteter Zustand des Knochens K gezeigt, wobei die Federsegmente FS1 und FS2 des jeweiligen Inselabschnitts IA eine Grundstellung des jeweiligen Inselabschnitts IA einstellen und sich hierdurch hinsichtlich des Spalts S zwischen den Knochenfragmenten K1 und K2 ein Spaltmaß SM1 ergibt. Dagegen zeigt Fig. 1B den Knochen K bei einer Belastung B, welche in Fig. 1B mit einem Pfeil angedeutet ist und eine übliche Beanspruchung des Knochens K darstellt. So kann es sich bei dieser Belastung B im Falle der bevorzugten Ausführung des Knochens K als Mandibula um eine abzustützende Beißkraft handeln.

Als Besonderheit verlaufen die Federsegmente FS1 und FS2 spitzwinkelig zu einem Kraftvektor KV, durch welchen eine an dem jeweiligen Befestigungspunkt BP2 aufgrund der Belastung B resultierende Querkraft repräsentiert ist und der in Fig. 1B für einen der Befestigungspunkte BP2 mit einem Pfeil angedeutet ist. Neben diesen - in Fig. 1B jeweils gestrichelt angedeuteten - spitzwinkeligen Verläufen zu dem Kraftvektor KV verlaufen die Federsegmente FS1 und FS2 des jeweiligen Inselabschnitts IA zudem divergierend zu dem Brückenabschnitt BU. Dies hat zur Folge, dass an den Federsegmenten FS1 und FS2 bei Einleitung der Belastung B in das Knochenfragment K2 eine Kraftumleitung stattfindet, bei welcher die Inselabschnitte IA von dem Brückenabschnitt BU wegbewegt werden und damit eine Distanz zwischen den Inselabschnitten IA und dem Brückenabschnitt BU vergrößert wird. Dies resultiert in einer Auseinanderbewegung der Knochenfragmente K1 und K2 und damit einer Vergrößerung des Spalts S auf ein Spaltmaß SM2.

Mit Beendigung der Belastung B verkleinert sich der Spalt S dann wieder auf das Spaltmaß SM1, indem die Federsegmente FS1 und FS2 des jeweiligen Inselabschnitts IA durch elastisches Zurückverformen eine entsprechende Rückverschiebung hervorrufen. Generell wird durch die Relativbewegung der Knochenfragmente K1 und K2 zueinander der Heilungsprozess des Knochens K begünstigt.

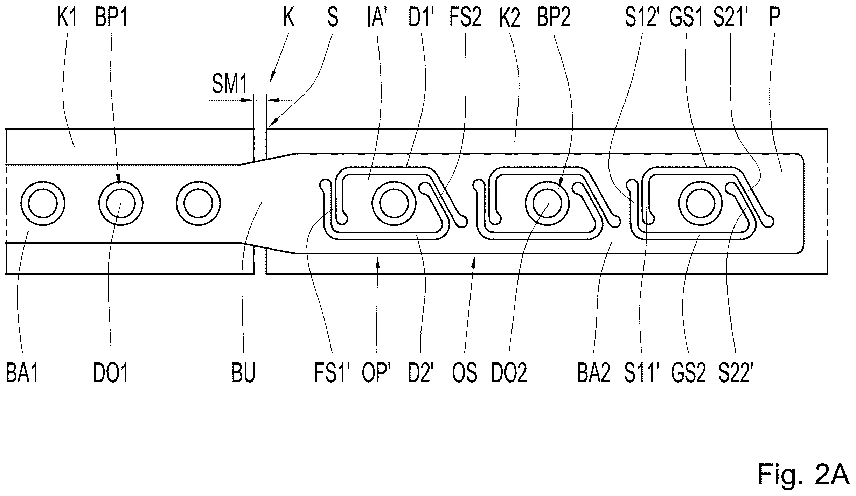

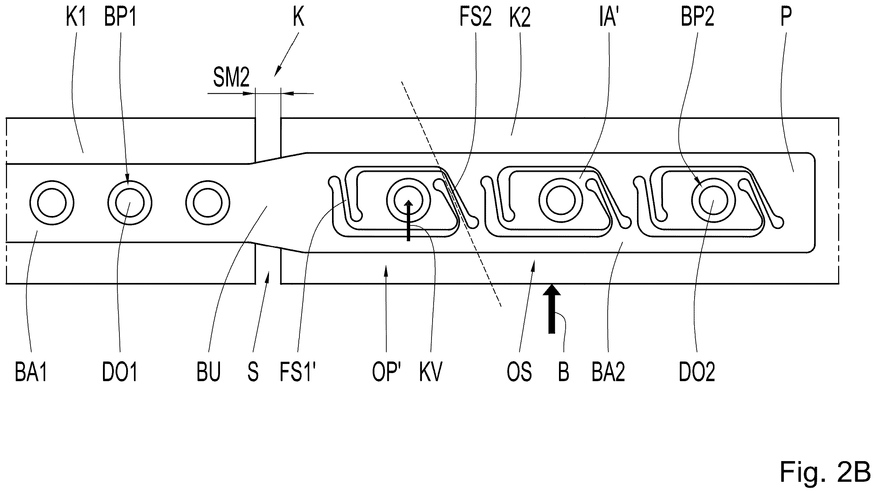

Des Weiteren zeigen die Fig. 2A und 2B schematische Ansichten des Knochens K, an welchem in diesem Fall eine Osteosyntheseplatte OP' entsprechend einer weiteren Ausgestaltungsmöglichkeit der Erfindung befestigt ist. Diese Osteosyntheseplatte OP' entspricht hierbei weitestgehend der Osteosyntheseplatte OP aus den Fig. 1A und 1B, wobei im Unterschied dazu nun nicht-symmetrisch ausgebildete Inselabschnitte IA' an dem Befestigungsabschnitt BA2 des Plattenkörpers P ausgestaltet sind. Dies ist hierbei dadurch realisiert, dass der jeweilige Inselabschnitt IA' und auch die zugehörigen Federsegmente FS1' und FS2 vorliegend durch Durchbrüche D1' und D2'gebildet sind, die zwar erneut U-förmig ausgeführt sind, sich dabei aber in ihrer jeweiligen U-Form voneinander unterscheiden. So weisen zwar beide U-Formen je einen ersten, quer zu der jeweiligen Grundseite GS1 bzw. GS2 der U-Form verlaufenden Schenkel S11' bzw. S12' auf, wobei dann aber bei der U-Form des Durchbruchs D1' ein zweiter Schenkel S21' gegenüber der Grundseite GS1 nach außen angewinkelt verläuft, wohingegen ein zweiter Schenkel S22' bei der U-Form des Durchbruchs D2' nach innen angewinkelt ist. Hierdurch ergeben sich lineare und asymmetrische Verläufe der Federsegmente FS1' und FS2 zu dem jeweiligen Befestigungspunkt BP2. Aufgrund dieser asymmetrischen Verläufe können die an dem Befestigungsabschnitt BA2 vorgesehenen Inselabschnitte IA' dicht aufeinanderfolgend angeordnet werden.

Während das jeweils eine Federsegment FS2 hierbei wie in der Ausbildung gemäß Fig. 1A und Fig. 1B spitzwinkelig verläuft, ergibt sich durch den asymmetrischen Aufbau ein annähernd orthogonaler Verlauf des Federsegments FS1' zu dem Befestigungsabschnitt BA2, wodurch sich in dem in Fig. 2B gezeigten Zustand bei Einleitung der Belastung B ein nahezu paralleler Verlauf des Federsegments FS1' zu dem Kraftvektor KV ergibt. Aufgrund der Ausrichtung des Federsegments FS2 im Verhältnis zum Kraftvektor KV ergibt sich dennoch eine Verschiebung des Inselabschnitts IA' relativ zum Befestigungsabschnitt BA2, sodass eine Distanz zwischen den Inselabschnitten IA' und dem Brückenabschnitt BU vergrößert wird.

Ansonsten entspricht die Ausgestaltung nach den Fig. 2A und 2B der vorhergehenden Variante nach den Fig. 1A und 1B, so dass auf das hierzu Beschriebene Bezug genommen wird.

Die Spaltmaße SM1, SM2 in Fig. 1A, Fig. 1B, Fig. 2A und Fig. 2B sowie die Auslenkung der Inselabschnitte IA in Fig. 1B und Fig. 2B sind zur besseren Illustration stark übertrieben dargestellt. Bei einer tatsächlichen Anwendung der Osteosyntheseplatte OP, OP' werden selbstverständlich kleinere Spaltmaße verwendet, und eine übliche Belastung B des Knochens K würde in einer geringeren Auslenkung der Inselabschnitte IA resultieren als in Fig. 1B und Fig. 2B dargestellt.

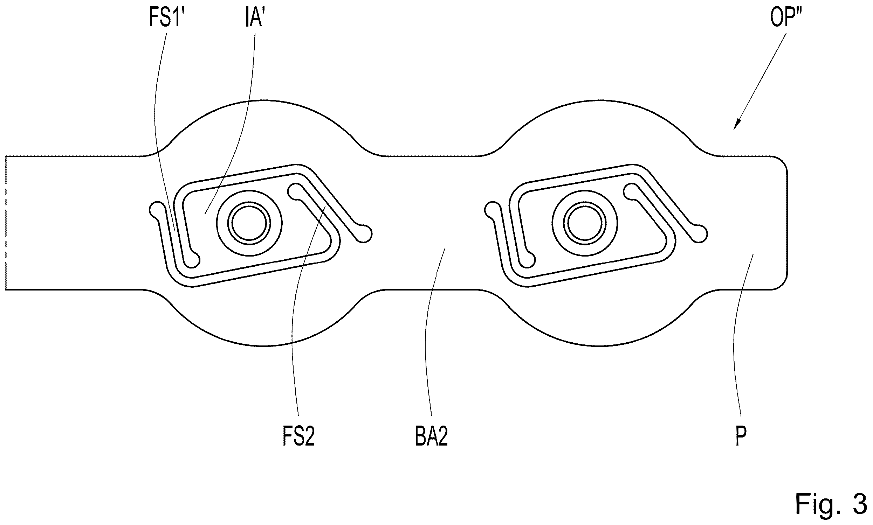

Aus Fig. 3 geht eine schematische Ansicht eines Teils einer Osteosyntheseplatte OP" entsprechend einer weiteren Ausführungsform der Erfindung hervor. Dabei entspricht diese Ausführungsform weitestgehend der vorhergehenden Variante nach den Fig. 2A und 2B, mit dem Unterschied, dass die Inselabschnitte IA' nun an dem gezeigten Befestigungsabschnitt BA2 des Plattenkörpers P jeweils um einen Kippwinkel im Gegenuhrzeigersinn verkippt definiert sind. Aufgrund dieses Verkippens ergibt sich bei den jeweiligen Federsegmenten FS1' und FS2 jeweils eine spitzwinkelige Ausrichtung zu dem jeweiligen - hier nicht gezeigten - Kraftvektor. Der Plattenkörper P weist im Bereich der Inselabschnitte IA' einen verbreiterten Querschnitt auf. Ansonsten entspricht die Ausführungsform nach Fig. 3 der Variante nach den Fig. 2A und 2B, so dass auf das hierzu Beschriebene Bezug genommen wird.

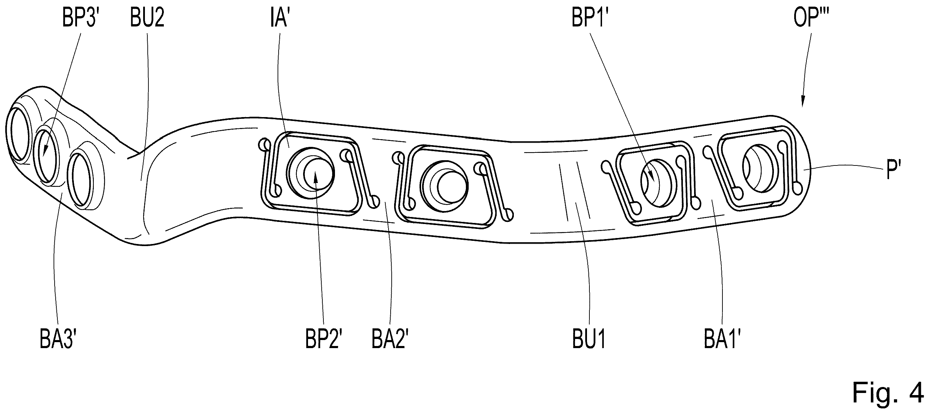

Ferner zeigt noch Fig. 4 eine perspektivische Darstellung einer Osteosyntheseplatte OP‴ entsprechend einer weiteren Ausgestaltungsmöglichkeit der Erfindung. Unterschiedlich gegenüber den vorstehend beschriebenen Varianten ist hierbei, dass ein Plattenkörper P' dieser Osteosyntheseplatte OP‴ hierbei zwei Brückenabschnitte BU1 und BU2 zum Überbrücken je eines, in Fig. 4 nicht dargestellten Spalts aufweist. Insofern können über die Osteosyntheseplatte OP‴ drei Knochenfragmente eines Knochens zu dessen Wiederherstellung zueinander stabilisiert werden. Die Brückenabschnitte BU1 und BU2 sind dabei zwischenliegend zu Befestigungsabschnitten BA1', BA2' und BA3' definiert, welche jeweils der Befestigung an je einem der Knochenfragmente dienen. Während Befestigungspunkte BP3' dabei an dem Befestigungsabschnitt BA3' ortsfest ausgestaltet sind, sind die Befestigungspunkte BP1' und BP2' bei den Befestigungsabschnitten BA1' und BA2' jeweils an Inselabschnitten IA' definiert, die hierbei in analoger Weise zu der Variante nach den Fig. 2A und 2B gestaltet sind. Insofern wird diesbezüglich auf das zu den Fig. 2A und 2B Beschriebene Bezug genommen.

Mittels der erfindungsgemäßen Ausgestaltungen kann jeweils eine Osteosyntheseplatte geschaffen werden, bei deren Verwendung bei der Wiederherstellung eines Knochens ein Heilungsprozess des Knochens auf zuverlässige Weise unterstützt wird.

### Bezugszeichenliste

- K: Knochen
- OP, OP', OP", OP‴: Osteosyntheseplatte
- K1, K2: Knochenfragmente
- P, P': Plattenkörper
- OS: Oberseite
- BA1, BA2, BA1', BA2, 'BA3': Befestigungsabschnitte
- BU, BU1, BU2: Brückenabschnitt
- BP1, BP2, BP1', BP2', BP3': Befestigungspunkte
- DO1, DO2: Durchgangsöffnungen
- S: Spalt
- IA, IA': Inselabschnitte
- FS1, FS2, FS1': Federsegmente
- D1, D2, D1', D2': Durchbrüche
- GS1, GS2: Grundseiten
- S11, S21, S11', S21': Schenkel
- S12, S22, S12', S22': Schenkel
- SM1, SM2: Spaltmaße
- B: Belastung
- KV: Kraftvektor

## Patentansprüche

1. Osteosyntheseplatte (OP, OP', OP", OP‴), welche für eine Fixierung von Knochenfragmenten (K1, K2) zur Wiederherstellung eines Knochens (K), insbesondere einer Mandibula, vorgesehen ist, umfassend einen Plattenkörper (P, P') mit einer Oberseite (OS) und einer Unterseite, auf welcher der Plattenkörper (P, P') an den Knochenfragmenten (K1, K2) aufzulegen ist, wobei der Plattenkörper (P, P') zumindest einen Brückenabschnitt (BU, BU1, BU2) aufweist, welcher einem Überbrücken eines zwischen den Knochenfragmenten (K1, K2) verlaufenden Spalts (S) dient, wobei der Plattenkörper (P, P') mit beidseitig des zumindest einen Brückenabschnitts (BU, BU1, BU2) liegenden Befestigungsabschnitten (BA1, BA2, BA1', BA2, 'BA3') ausgestattet ist, an welchen auf der Unterseite Befestigungen der Osteosyntheseplatte (OP, OP', OP", OP‴) seitens der Knochenfragmente (K1, K2) vorzunehmen sind und denen dazu jeweils mindestens ein Befestigungspunkt (BP1, BP2, BP1', BP2', BP3') zugeordnet ist, wobei der mindestens eine Befestigungspunkt (BP2, BP1', BP2') bei zumindest einem der Befestigungsabschnitte (BA1, BA2, BA1', BA2, 'BA3') an einem Inselabschnitt (IA, IA') ausgestaltet ist, welcher mit dem jeweils zugehörigen Befestigungsabschnitt (BA2, BA1', BA2') ausschließlich über Federsegmente (FS1, FS2, FS1', FS2) verbunden ist, welche eine Relativverschiebung des jeweiligen Befestigungsabschnitts (BA2, BA1', BA2') und des jeweiligen Inselabschnitts (IA, IA') zueinander elastisch federnd zulassen, **dadurch gekennzeichnet, dass** zumindest eines der Federsegmente (FS1, FS2, FS1', FS2) so ausgerichtet ist, dass bei einer Belastung (B) in einer Hauptbelastungsrichtung des Knochens (K) eine Relativverschiebung zwischen dem Inselabschnitt (IA, IA') und dem diesem Inselabschnitt (IA, IA') zugeordneten Befestigungsabschnitt (BA2, BA1', BA2') stattfindet, bei welcher sich eine Distanz des Inselabschnitts (IA, IA') zu dem zumindest einen Brückenabschnitt (BU, BU1) vergrößert.

2. Osteosyntheseplatte (OP, OP', OP", OP‴) nach Anspruch 1, **dadurch gekennzeichnet, dass** das zumindest eine Federsegment (FS1, FS2, FS2, FS1', FS2) spitzwinkelig zu einem Kraftvektor (KV) ausgerichtet ist, welcher eine Querkraft repräsentiert, die an dem zugehörigen Befestigungspunkt (BP2, BP1', BP2') des Inselabschnitts (IA, IA') bei der Belastung (B) des Knochens (K) quer zu dem jeweiligen Befestigungsabschnitt (BA2, BA1', BA2') wirkend resultiert.

3. Osteosyntheseplatte (OP, OP', OP", OP‴) nach Anspruch 1 oder 2, **dadurch gekennzeichnet, dass** der Inselabschnitt (IA, IA') über je zwei Federsegmente (FS1, FS2, FS1', FS2) mit dem jeweiligen Befestigungsabschnitt (BA2, BA1', BA2') verbunden ist.

4. Osteosyntheseplatte (OP, OP', OP", OP‴) nach Anspruch 3, **dadurch gekennzeichnet, dass** der Inselabschnitt (IA, IA') und die beiden zugehörigen Federsegmente (FS1, FS2, FS1', FS2) durch zwei U-förmige Durchbrüche (D1, D2, D1', D2') gebildet sind, welche mit ihren Öffnungen einander zugewandt und ineinander geschachtelt an dem jeweiligen Befestigungsabschnitt (BA2, BA1', BA2') ausgestaltet sind.

5. Osteosyntheseplatte (OP) nach Anspruch 3 oder Anspruch 4, **dadurch gekennzeichnet, dass** die Federsegmente (FS1, FS2) den Inselabschnitt (IA) symmetrisch verlaufend, insbesondere punktsymmetrisch zu dem jeweiligen Befestigungspunkt (BP2), mit dem jeweiligen Befestigungsabschnitt (BA2) verbinden.

6. Osteosyntheseplatte (OP', OP", OP‴) nach Anspruch 3 oder Anspruch 4, **dadurch gekennzeichnet, dass** die Federsegmente (FS1', FS2) den Inselabschnitt (IA') asymmetrisch verlaufend mit dem Plattenkörper (P, P') verbinden.

7. Osteosyntheseplatte (OP, OP', OP", OP‴) nach wenigstens einem der vorhergehenden Ansprüche, **dadurch gekennzeichnet, dass** dem jeweiligen Befestigungsabschnitt (BA2, BA1', BA2') mehrere Befestigungspunkte (BP2, BP1', BP2') zugeordnet sind, wobei jeder der Befestigungspunkte (BP2, BP1', BP2') an jeweils einem Inselabschnitt (IA, IA') ausgestaltet ist.

8. Osteosyntheseplatte (OP, OP', OP", OP‴) nach Anspruch 7, **dadurch gekennzeichnet, dass** bei mehreren oder bei allen Inselabschnitten (IA, IA') der mehreren Befestigungspunkte (BP2, BP1', BP2') jeweils zumindest eines der jeweils zugeordneten Federsegmente (FS1, FS2, FS2, FS1', FS2) die Distanz bei der Belastung (B) vergrößernd ausgerichtet ist.

9. Osteosyntheseplatte (OP, OP') nach wenigstens einem der vorhergehenden Ansprüche, **dadurch gekennzeichnet, dass** von den beidseitig des zumindest einen Brückenabschnitts (BU) liegenden Befestigungsabschnitten (BA1, BA2) der mindestens eine Befestigungspunkt (BP2) bei dem einen Befestigungsabschnitt (BA2) an dem jeweiligen Inselabschnitt (IA, IA') ausgestaltet ist, wohingegen der mindestens eine Befestigungspunkt (BP1) bei dem anderen Befestigungsabschnitt (BA1) ortsfest an dem anderen Befestigungsabschnitt (BA1) ausgebildet ist.

10. Osteosyntheseplatte (OP‴) nach wenigstens einem der Ansprüche 1 bis 8, **dadurch gekennzeichnet, dass** die Befestigungspunkte (BP1', BP2) beidseitig des zumindest einen Brückenabschnitts (BU1) an Inselabschnitten (IA') ausgestaltet sind.

11. Osteosyntheseplatte (OP‴) nach Anspruch 10, **dadurch gekennzeichnet, dass** bei den Inselabschnitten (IA') der beidseitig des Brückenabschnitts (BU1) angeordneten Befestigungsabschnitte (BA1', BA2) jeweils zumindest eines der zugeordneten Federsegmente (FS1', FS2) die Distanz bei der Belastung (B) vergrößernd ausgerichtet ist.

12. Osteosyntheseplatte (OP, OP', OP") nach wenigstens einem der vorhergehenden Ansprüche, **dadurch gekennzeichnet, dass** der Plattenkörper (P) genau einen Brückenabschnitt (BU) aufweist.

13. Osteosyntheseplatte (OP‴) nach wenigstens einem der Ansprüche 1 bis 11, **dadurch gekennzeichnet, dass** der Plattenkörper (P') mehrere Brückenabschnitte (BU1, BU2) zur Überbrückung mehrerer Spalte (S) aufweist, wobei jeweils beidseitig des jeweiligen Brückenabschnitts (BU1, BU2) Befestigungsabschnitte (BA1', BA2, 'BA3') vorgesehen sind.
